(19) Europäisches Patentamt
European Patent Office
Office européen des brevets

(11) Veröffentlichungsnummer: **0 004 332**
B1

(12) # EUROPÄISCHE PATENTSCHRIFT

(45) Veröffentlichungstag der Patentschrift: **26.08.81**

(21) Anmeldenummer: **79100737.0**

(22) Anmeldetag: **12.03.79**

(51) Int. Cl.³: **C 07 D 403/04,** C 07 D 403/06, C 07 D 405/02, C 07 D 409/04, C 07 D 217/22, C 07 D 217/26, A 61 K 31/47

(54) **Isochinole,Verfahren zu ihrer Herstellung und diese enthaltende Arzneimittel.**

(30) Priorität: **16.03.78 DE 2811312**

(43) Veröffentlichungstag der Anmeldung: **03.10.79 Patentblatt 79/20**

(45) Bekanntmachung des Hinweises auf die Patenterteilung: **26.08.81 Patentblatt 81/34**

(84) Benannte Vertragsstaaten: **CH DE FR GB**

(56) Entgegenhaltungen: **AU-B-49 934/72**

(73) Patentinhaber: **HOECHST AKTIENGESELLSCHAFT, Zentrale Patentabteilung Postfach 80 03 20, D-6230 Frankfurt/Main 80 (DE)**

(72) Erfinder: **Bartmann, Wilhelm, Dr., Am Dachsbau 5, D-6232 Bad Soden am Taunus (DE)**
Erfinder: **Konz, Elmar, Dr., Buchenweg 22, D-6232 Bad Soden am Taunus (DE)**
Erfinder: **Kruse, Hansjörg, Dr., Feldbergstrasse 70, D-6233 Kelkheim (Taunus) (DE)**
Erfinder: **Geyer, Harry Maurice, Dr., Feldbergstrasse 70, D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



EP 0 004 332 B1

# Isochinoline, Verfahren zu ihrer Herstellung und diese enthaltende Arzneimittel

Die Erfindung betrifft in 3-Stellung basisch substituierte Isochinoline, die wertvolle pharmakologische, insbesondere psychotrope Eigenschaften besitzen.

3-Amino-4-phenyl-isochinolinderivate mit Wirkungen auf das Zentralnervensystem werden in der DE-OS 2 030 675 und 1-Amino-isochinoline mit fungizider und rodentizider Wirkung in der DE-OS 2 243 789 beschrieben.

Gegenstand der Erfindung sind Isochinoline der Formel I

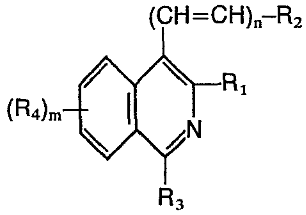

(I)

und deren physiologisch verträgliche Salze, worin bedeuten:

m eins oder zwei

n null oder eins

$R_1$ eine Aminogruppe der Formel

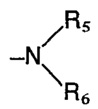

,

worin $R_5$ und $R_6$ gleich oder verschieden sind und Wasserstoff oder Alkylreste mit 1 bis 8 Kohlenstoffatomen bedeuten, wobei die Alkylreste auch substituiert sein können mit Hydroxy-, $C_1$–$C_4$-Alkoxy oder eine Aminogruppe der Formel

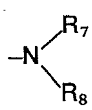

wobei $R_7$ und $R_8$ gleich oder verschieden sind und Wasserstoff oder einen geradkettigen oder verzweigten Alkylrest mit 1 bis 6 Kohlenstoffatomen, oder zusammen mit dem Stickstoffatom einen heterocyclischen Ring mit bis zu 7 Kohlenstoffatomen darstellen. Die Alkylreste $R_5$ und $R_6$ können auch gemeinsam mit dem Stickstoffatom einen 5- bis 8gliedrigen Ring bilden, wobei der heterocyclische Ring an einem Kohlenstoffatom substituiert sein kann mit einer $C_1$–$C_6$-Alkyl-, $C_1$–$C_4$-Alkoxy-, Hydroxy-, Carboxy- oder $C_1$–$C_4$-Alkoxycarbonylgruppe, und worin eines der Kohlenstoffatome durch ein Sauerstoff-, Schwefel- oder Stickstoffatom ersetzt sein kann, wobei das letztere substituiert sein kann durch Wasserstoff, die Thienyl-, Furyl-, Pyridyl- oder Formylgruppe, eine $C_3$–$C_8$-Alkenyloxycarbonyl- oder $C_3$–$C_8$-Alkinyloxycarbonylgruppe, eine gegebenenfalls durch Hydroxy- oder $C_1$–$C_4$-Alkoxygruppen substituierte $C_1$–$C_6$-Alkoxycarbonylgruppe; durch einen Phenylrest, der einfach oder mehrfach substituiert sein kann mit $C_1$–$C_4$-Alkyl-, $C_1$–$C_4$-Alkoxy, Methylendioxy-, Hydroxy-, Nitro- oder Aminogruppe oder Halogen; durch den Rest $-COR_9$, worin $R_9$ einen Thienyl-, Furyl-, Pyridylrest oder einen gegebenenfalls wie oben angegebenen substituierten Phenylrest; oder durch eine $C_1$–$C_6$-Alkylgruppe, die ihrerseits substituiert sein kann mit Hydroxy, $C_1$–$C_4$-Alkoxy-, $C_1$–$C_6$-Dialkylamino, die Äthylendioxy- oder die Methylendioxygruppe oder einen gegebenenfalls wie oben angegebenen substituierten Phenylrest;

$R_2$ eine Carboxyl-, Cyano-, Formyl-, Hydroxymethyl-, eine Alkoxymethylgruppe mit 1 bis 6 Kohlenstoffatomen, eine Aminoalkylgruppe der Formel

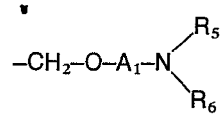

,

worin $A_1$ eine geradkettige oder verzweigte $C_2$–$C_6$-Alkylengruppe darstellt, die durch Hydroxy oder $C_1$–$C_4$-Alkoxygruppen substituiert sein kann und worin $R_5$ und $R_6$ die oben genannte Bedeutung haben, eine Acyloxymethylgruppe der Formel $-CH_2-O-CO-R_{10}$, worin $R_{10}$ eine $C_1$–$C_6$-Alkyl-, oder einen Phenylrest bedeutet, der gegebenenfalls wie oben angegeben substituiert sein kann, eine Aminomethylgruppe der Formel

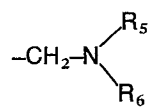

,

worin $R_5$ und $R_6$ die obengenannte Bedeutung haben, eine Carbonamidgruppe der Formel

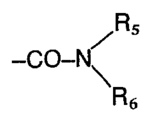

,

worin $R_5$ und $R_6$ die oben genannte Bedeutung haben, oder eine Carbonestergruppe der Formel

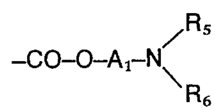

,

worin $A_1$, $R_5$ und $R_6$ die oben genannte Bedeutung haben,

$R_3$ ein Phenylrest der gegebenenfalls mit Halogen-, Hydroxy-, Nitro-, Amino- oder einer durch ein oder zwei aliphatische Kohlenwasserstoffreste substituierten Aminogruppe mit zwei bis achtzehn Kohlenstoffatomen, einer Acylamino-, Alkyl- oder Alkoxygruppe mit jeweils einem bis sechs Kohlenstoffatomen, einer Benzyloxy- oder einer Trifluormethylgruppe mono- oder disubstituiert ist, eine Pyridyl- oder Thienylrest;

$R_4$ Wasserstoff, Halogen, Hydroxy-, Alkyl- oder Alkylgruppe mit einem bis sechs Kohlenstoffatomen, Nitro-, Amino-, Benzyloxy-, Methylendioxy-, oder Äthylendioxygruppe, Verfahren zur Herstellung der Verbindungen und diese enthaltende Arzneimittel.

Insbesondere beinhaltet die Erfindung Verbindungen, worin $R_1$ eine Aminogruppe der Formel

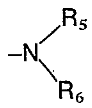 ,

ist, worin $R_5$ und $R_6$ gleich oder verschieden sind und Wasserstoff, einen geradkettigen oder verzweigten Alkylrest mit 1 bis 4 Kohlenstoffatomen darstellen, wobei die Alkylreste auch gemeinsam mit dem Stickstoffatom einen 5- bis 7gliedrigen Ring bilden können, worin eins der Kohlenstoffatome durch ein Sauerstoff-, Schwefel- oder Stickstoffatom ersetzt sein kann, wobei das letztere substituiert sein kann durch Wasserstoff, den Thienyl-, Furyl-, Pyridyl- oder Formylgruppe, eine gegebenenfalls durch Hydroxy- oder $C_1$–$C_4$-Alkoxygruppen substituierte $C_1$–$C_4$-Alkoxycarbonylgruppe, durch den Phenylrest der einfach oder mehrfach substituiert sein kann mit $C_1$–$C_4$-Alkyl-, $C_1$–$C_4$-Alkoxy-, Methylendioxy-, Hydroxy-, Nitro-, Amino- oder Halogen; durch den Rest $-COR_9$, worin $R_9$ einen Thienyl-, Furyl-, Pyridylrest oder einen gegebenenfalls wie oben angegebenen substituierten Phenylrest bedeutet, oder durch eine $C_{11}$–$C_4$-Alkylgruppe die ihrerseits substituiert sein kann mit Hydroxy, $C_1$–$C_4$-Alkoxy-, $C_1$–$C_4$-Dialkylamino oder dem gegebenenfalls wie oben angegebenen substituierten Phenylrest, worin weiterhin, wenn $R_5$ Wasserstoff oder $C_1$–$C_4$-Alkyl ist, $R_6$ einen Aminoalkylrest der Formel

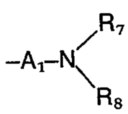

bedeutet, worin $A_1$, $R_7$ und $R_8$ die oben angegebene Bedeutung haben. Die bevorzugten Substituenten für $R_2$ sind eine Carboxyl-, Cyano-, Formyl-, Hydroxymethyl-, eine Alkoxymethylgruppe mit 1 bis 4 Kohlenstoffatomen oder ein Aminoalkylrest der Formel

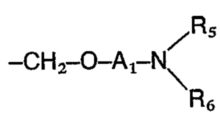

worin $A_1$, $R_5$ und $R_6$ die oben genannte Bedeutung haben, oder eine Methylaminogruppe der Formel

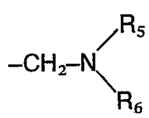

worin $R_5$ und $R_6$ die oben genannte Bedeutung haben, während die bevorzugten Substituenten für $R_3$ ein gegebenenfalls mit Halogen, Nitro, Alkyl- oder Alkoxy mit 1 bis 4 Kohlenstoffatomen oder Amino mono- oder disubstituierter Phenylring und für $R_4$ Wasserstoff, Halogen, Hydroxy, Nitro-, Amino, Alkyl oder Alkoxy mit 1 bis 4 Kohlenstoffatomen sind.

Von ganz besonderem Interesse sind Verbindungen, worin n gleich Null ist, $R_1$ einen Aminorest der Formel

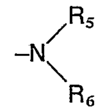

in dem die Alkylreste $R_5$ und $R_6$ gemeinsam mit dem Stickstoffatom einen 5- bis 7gliedrigen Ring bilden und worin eines der Kohlenstoffatome durch ein N- oder O-Atom ersetzt sein kann, insbesondere der Pyrrolidino-, Piperidino-, Hexamethylenimino-, Morpholino-, 4-Hydroxypiperidino-, 4-Carbäthoxypiperidino- und der 1-Piperazinylrest

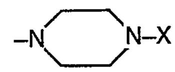 ,

worin X Wasserstoff, $C_1$–$C_4$-Alkyl, $\beta$-Hydroxyäthyl, 3,4-Methylendioxybenzyl, Phenyl, durch Methoxy, Chlor, Nitro oder Amino substituiertes Phenyl, 3,4,5-Trimethoxybenzoyl, 3,4-Methylendioxybenzoyl, 2-Furoyl, 2-Thienoyl, $C_1$–$C_3$-Alkoxycarbonyl bedeutet, wobei der Alkylrest im letzteren durch OH oder Methoxy und Äthoxy substituiert sein kann, oder wenn $R_5$ Wasserstoff oder $C_1$–$C_4$ Alkyl ist, $R_6$ einen Aminoalkylrest der Formel

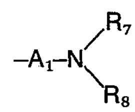

worin $A_1$, $R_7$ und $R_8$ die oben genannte Bedeutung haben. Für $R_2$ kommt der Carboxyl-, Cyano-, Formyl- oder Hydroxymethylgruppe für $R_3$ dem Phenylrest, der gegebenenfalls mit Halogen, Hydroxy-, Nitro-, Amino- oder Methoxygruppen mono- oder disubstituiert ist, und für $R_4$ Wasserstoff, Halogen, Hydroxy oder Methoxygruppen, bevorzugt in 6- und/oder 7-Stellung ganz besondere Bedeutung zu. Gegenstand der Erfindung sind weiterhin Verfahren zur Herstellung sowie pharmazeutische Zubereitungen dieser Verbindungen.

Das Verfahren zur Herstellung der Verbindungen der Formel I ist dadurch gekennzeichnet, dass man

$a_1$) Verbindungen der allgemeinen Formel II

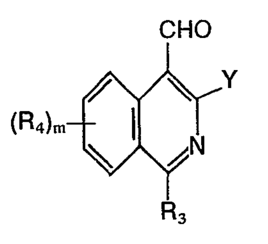 (II)

worin Y Chlor oder Brom und $R_3$, $R_4$ und m die zur Formel I genannte Bedeutung haben, mit einem Amin der Formel

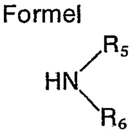 ,

worin $R_5$ und $R_6$ die zur Formel I genannte Bedeutung haben, zu den Verbindungen der Formel I umsetzt, worin n gleich Null ist und $R_2$ eine Formylgruppe darstellt. Diese können gegebenenfalls durch Umsetzung mit Verbindungen der Formel IIIa oder IIIb,

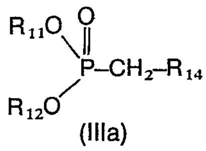

(IIIa)

$(R_{13})_3\overset{\oplus}{P}-CH_2-R_{14}$ $Br^{\ominus}$

(IIIb)

worin $R_{11}$, $R_{12}$ und $R_{13}$ eine $C_1$–$C_4$-Alkylgruppe oder den Phenylrest bedeuten und $R_{14}$ vorzugsweise eine Nitrilgruppe ist, zu den Verbindungen der Formel I überführt werden, in denen n = 1 ist,

$a_2$) Verbindungen der Formel IV

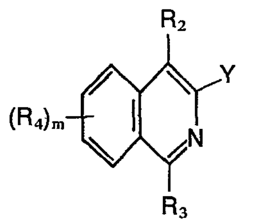

(IV)

worin Y Chlor oder Brom, $R_2$ eine Cyano oder Carbonamidgruppe der Formel

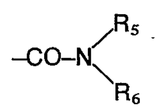

und $R_3$, $R_4$, $R_5$, $R_6$ und m die zur Formel I genannte Bedeutung haben, mit einem Amin der Formel

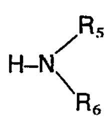

,

worin $R_5$ und $R_6$ die zur Formel I genannte Bedeutung haben, zu den Verbindungen der Formel I umsetzt.

b) Verbindungen der Formel V

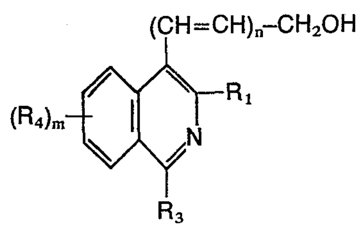

(V)

worin $R_1$, $R_3$, $R_4$, n und m die zur Formel I genannte Bedeutung haben zu Verbindungen der Formel I oxidiert, worin $R_2$ eine Formylgruppe ist,

c) Verbindungen der Formel VI

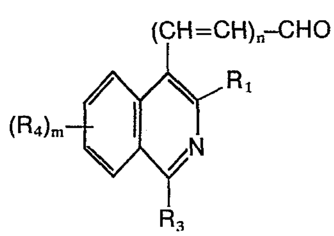

(VI)

worin $R_1$, $R_3$, $R_4$, m und n die zur Formel I genannte Bedeutung haben zu den Verbindungen der Formel I oxydiert, worin $R_2$ eine Carboxylgruppe ist. Diese können gegebenenfalls mit einem Amin

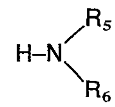

,

oder mit einem Alkohol der Formel

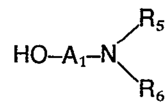

,

worin $R_5$, $R_6$ und $A_1$ die zur Formel I genannte Bedeutung haben, zu Verbindungen der Formel I umgesetzt werden,

d) Verbindungen der Formel VI zu Verbindungen der Formel I reduziert, worin $R_2$ eine Hydroxymethylgruppe ist,

e) Verbindungen der Formel VII

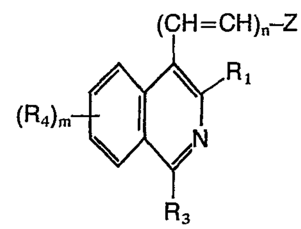

(VII)

worin Z eine Nitril-, Carboxyl-, Halogencarboxyl- oder eine Alkylcarboxylgruppe mit 1 bis 7 Kohlenstoffatomen bedeutet und $R_1$, $R_3$, $R_4$, m und n die zur Formel I genannte Bedeutung haben, zu Verbindungen der Formel I reduziert, worin $R_2$ eine Formylgruppe darstellt.

f) Verbindungen der Formel VIII

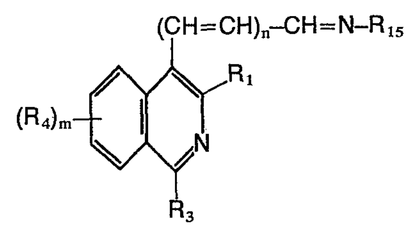

(VIII)

worin $R_1$, $R_3$, $R_4$, m und n die zur Formel I genannte Bedeutung haben, zu Verbindungen der Formel I reduziert, worin $R_2$ die Methylenaminogruppe der Formel $-CH_2-NH-R_{15}$ und $R_{15}$ der Rest

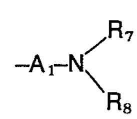

sein soll, wobei $A_1$, $R_7$ und $R_8$ die zur Formel I genannte Bedeutung haben;

g) Verbindungen der Formel IX

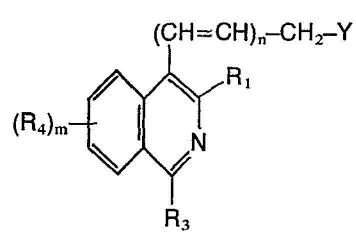

(IX)

worin Y gleich Chlor oder Brom ist, mit einem Amin der Formel

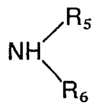

oder einem Alkohol der Formel

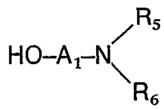

umsetzt, wobei in den obigen Formeln m, n, $R_1$, $R_3$ bis $R_6$ und $A_1$ die im Anspruch 1 genannte Bedeutung haben;

h) Verbindungen der Formel X

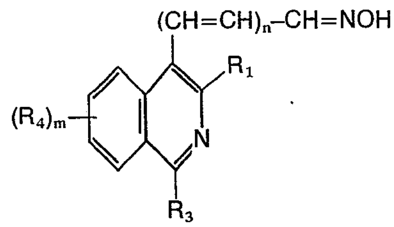

(X)

worin $R_1$, $R_3$, $R_4$, m und n die zur Formel I genannte Bedeutung haben, mit wasserabspaltenden Mitteln in Verbindungen der Formel I überführt, worin $R_2$ eine Nitrilgruppe darstellt;

i) Verbindungen der Formel XI

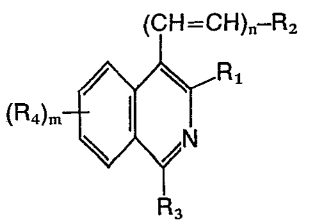

(XI)

worin $R_2$ bis $R_4$, m und n die zur Formel I genannten Bedeutungen haben und $R_1$ ein Piperazinylring ist, mit einem Alkylierungsmittel der Formel $Y–R_{16}$, worin Y Chlor oder Brom und $R_{16}$ einen geradkettigen oder verzweigten $C_1$–$C_6$-Alkylrest bedeuten, der durch Hydroxy, $C_1$–$C_4$-Alkoxy, $C_1$–$C_4$-Dialkylamino, Äthylendioxy, Methylendioxy, Phenyl oder eine oder mehrere $C_1$–$C_4$-Alkyl-, $C_1$–$C_4$-Alkoxy-, Methylendioxy-, Hydroxy-, Nitro- oder Aminogruppen oder Halogen tragendes Phenyl substituiert sein kann, oder mit einem Chlorameisensäureester der Formel Cl–COO–($C_1$–$C_4$)-alkyl oder mit einer Verbindung der Formel Cl–$COR_9$, in der $R_9$ die in Anspruch 1 genannte Bedeutung hat, umsetzt.

Bei der Verfahrensweise $a_1$) oder $a_2$) wird mindestens die doppelt äquivalente Menge Amin zugegeben, da ein Mol Amin zur Bindung des abgespaltenen Halogenwasserstoffs gebraucht wird, jedoch ist es manchmal von Vorteil, zur Reaktionsbeschleunigung einen bis zu 15fachen Überschuss des Amins anzuwenden. Arbeitet man mit äquimolaren Mengen Amin, so kann man tertiäre Amine, wie Triäthylamin, Pyridin oder Kaliumcarbonat als Säurefänger hinzugeben.

Als Lösungsmittel kommen, sofern sie zur Umsetzung verwendet werden, indifferente, wasserfreie, organische Lösungsmittel wie Dioxan, 1,2-Dimethoxyäthan, Diäthylenglykoldimethyläther, Diäthylenglykoldibutyläther, Benzol, Toluol, Xylol, Chlorbenzol, Dichlorbenzol, Trichlorbenzol, N,N-Dimethylformamid, Dimethylsulfoxid oder Hexamethylphosphorsäuretriamid in Frage. Die Reaktion wird im allgemeinen bei einer Temperatur zwischen 50 und 200° vorzugsweise zwischen 80 und 180°C ausgeführt. Die Umsetzung der Aldehyde mit den Verbindungen IIIa oder IIIb kann unter den für die Wittig- bzw. Horner-Reaktion gebräuchlichen Bedingungen durchgeführt werden. So kann z. B. die Reaktion der Phosphonate III a in Äther bei Raumtemperatur vorgenommen werden. Als Äther kommen bevorzugt in Betracht Diäthyläther, Tetrahydrofuran und Dimethoxyäthan. Das Phosphonat kann äquimolar oder im Überschuss eingesetzt werden. Die Reaktion ist gewöhnlich nach 3 bis 24 Stunden bei Temperaturen zwischen 10 und 50°C beendet. Einzelheiten über die Durchführung dieser Reaktion sind im J. Amer. Chem. Soc. 83, 1733 (1961) beschrieben. Einzelheiten für die Durchführung der Wittig-Reaktion sind in J. Org. Chem. 28, 1128 (1963) beschrieben.

Die Ausgangsverbindungen II für das Verfahren a) können gemäss der Europäischen Patentanmeldung Nr. 79100712.3 hergestellt werden z. B. durch Umsetzung von Verbindungen der Formel IIa

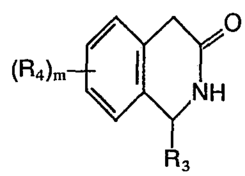

(IIa)

worin $R_3$, $R_4$ und m die für Formel I genannte Bedeutung haben mit einem Vilsmeier-Addukt aus einem Säureamid mit einem Säurechlorid zu Verbindungen der Formel IIb

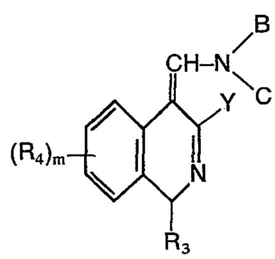

(IIb)

worin Y = Chlor oder Brom ist und B und C Alkyl oder Cycloalkyl mit einem bis sechs Kohlenstoffatomen oder Phenyl bedeuten und anschliessende Oxidation zu Verbindungen der Formel II.

Bei dem Verfahren b) werden die Verbindungen V nach bekannten Methoden, z. B. mit Mangandioxyd, oxydiert. Derartige Oxydationsreaktionen sind bekannt (vgl. z. B. «Compendium of Organic Synthetic Methods», Verlag John Wiley u. Sons, Inc. (1971) Seiten 146–147, 150–152).

Bei dem Verfahren c) werden die Verbindungen VI nach bekannten Methoden, z. B. mit Mangandioxyd, Kaliumpermanganat, oxydiert. Auch derartige Oxydationsreaktionen sind bekannt (vgl. «Compendium of Organic Synthetic Methods», Verlag John Wiley u. Sons, Inc. (1971), Seiten 32–36). Die Carbonsäuren werden nach den üblichen Methoden der Ester oder Amidbildung, z. B. über die Säurechloride, oder die gemischten Anhydride in die Ester bzw. Amide übergeführt. Zur Veresterung mit Alkoholen, die noch sekundäre Aminogruppen enthalten, werden die Salze der Aminoalkohole eingesetzt.

Bei dem Verfahren d) werden die Verbindungen VI nach bekannten Methoden reduziert. Als Reduktionsmittel kommen komplexe Metallhydride, z. B. Natriumborhydrid, Lithiumaluminiumhydrid und als Lösungsmittel Methanol, Äthanol, Tetrahydrofuran, Dimethoxyäthan in Betracht.

Das Verfahren e) beinhaltet auch eine prinzipiell bekannte Methode, nach der Verbindungen der Formel VII in eine erfindungsgemässe Verbindung der Formel I mit einer Formylgruppe für $R_2$ umgesetzt werden. Neben der Carboxylgruppe selbst kommen als geeignete Carbonsäurederivate, z. B. Ester, Säurechloride, Säureanhydride, Säureamide, Säureanhydride oder Nitrile und als Reduktionsmittel, z. B. komplexe Metallhydride wie Lithiumaluminiumhydrid, Diisobutylaluminiumhydrid, in Frage (vgl. «Compendium of Organic Synthetic Methods», Verlag John Wiley u. Sons, Inc. (1971), Seiten 132–137, 148–150, 152–153 und 166–168).

Bei dem Verfahren f) werden die Verbindungen VIII, nach bekannten Methoden reduziert. Als Reduktionsmittel kommen komplexe Metallhydride wie Natriumborhydrid, Lithiumaluminiumhydrid und als Lösungsmittel Methanol, Äthanol, Tetrahydrofuran, Dimethoxyäthan in Frage. Die Verbindungen VIII können aus den Verbindungen der Formel I mit einer Aldehydfunktion als $R_2$ durch Umsetzung mit einem Amin unter Säurekatalyse erhalten werden. Dabei kann das entstehende Reaktionswasser vorzugsweise mittels eines Wasserabscheiders abgetrennt werden.

Bei dem Verfahren g) werden die Verbindungen IX mit einem Amin oder Alkohol in Gegenwart eines Halogenwasserstoff bindenden Mittels umsetzt. Als Halogenwasserstoff bindende Mittel kommen ein Überschuss des Amins selbst, tertiäre Amine wie Triäthylamin, Pyridin, oder auch Kaliumcarbonat in Frage und als Lösungsmittel, sofern sie zur Umsetzung verwendet werden, können indifferente, wasserfreie, organische Lösungsmittel wie Dioxan, 1,2-Dimethoxyäthan, Diäthylenglykoldimethyläther, Diäthylenglykoldibutyläther, Benzol, Toluol, Xylol, Chlorbenzol, N,N-Dimethylformamid, Dimethylsulfoxid oder Hexamethylphosphorsäuretriamid eingesetzt werden. Die Reaktion wird im allgemeinen bei einer Temperatur zwischen 20 und 180 °C, vorzugsweise zwischen 20 und 130 °C ausgeführt. Die für das Verfahren g) benötigten Verbindungen II können durch Umsetzung der Verbindungen V mit Halogenierungsmitteln, wie Phosphortrichlorid, Phosphortribromid, nach bekannten Methoden dargestellt werden.

Bei dem Verfahren h) kommen als wasserabspaltende Mittel z. B. Phosphorpentoxyd, Phosphoroxychlorid, Essigsäureanhydrid in Frage. Die Reaktion wird im allgemeinen bei einer Temperatur zwischen 50 und 150 °C ausgeführt und als Lösungsmittel können Pyridin, Benzol, Toluol und N,N-Dimethylformamid verwendet werden.

Nach dem Verfahren i) wird der Piperazinring nach an sich bekannten Methoden mit Alkylierungsmittel $Y–R_{16}$ alkyliert oder mit Chlorameisensäureester oder $Cl–COR_9$ umgesetzt.

Die erfindungsgemässen Verbindungen haben wertvolle therapeutische Eigenschaften. So zeigen sie neben anderen pharmakologischen Eigenschaften eine Wirkung auf das Zentralnervensystem. Sie können die durch den elektrischen Strom oder Pentamethylentetrazol ausgelösten Krämpfe verhindern, verlängern die Thiopental- bzw. Alkoholnarkose, potenzieren aber andererseits die durch Isonicotinsäurehydrazid und Picrotoxin ausgelösten Konvulsionen. Aufgrund all dieser Eigenschaften können die erfindungsgemässen Verbindungen als Wirkstoffe von sedierend, tranquillisierend und krampfhemmend wirkenden Arzneimitteln angewandt werden.

Die neuen Verbindungen können entweder allein oder mit physiologisch verträglichen Hilfs- oder Trägerstoffen vermischt angewandt werden. Für eine orale Anwendungsform werden die aktiven Verbindungen mit den dafür üblichen Substanzen vermischt und durch übliche Methoden in geeigneten Darreichungsformen gebracht, wie Tabletten, Steckkapseln, wässrige, alkoholische oder ölige Suspensionen oder wässrige alkoholische oder ölige Lösungen. Als inerte Träger können z. B. Magnesiumcarbonat, Milchzucker oder Maisstärke verwendet werden. Dabei kann die Zubereitung sowohl als Trocken- oder Feuchtgranulat erfolgen. Als ölige Trägerstoffe oder Lösungsmittel kommen beispielsweise besonders pflanzliche und tierische Öle in Betracht wie Sonnenblumenöl oder Lebertran.

Eine besondere Anwendungsform liegt in der intravenösen Applikation. Zu diesem Zweck werden die aktiven Verbindungen oder deren physiologisch verträglichen Salze mit den dafür üblichen Substanzen in Lösung gebracht. Solche physiologisch verträglichen Salze werden z. B. mit folgenden Säuren gebildet: Chlor-, Brom- oder Jodwasserstoffsäure, Phosphorsäure, Schwefelsäure, Methylschwefelsäure, Amidosulfonsäure, Salpetersäure, Ameisensäure, Essigsäure, Propionsäure, Bernsteinsäure, Weinsäure, Milchsäure, Malonsäure, Fumarsäure, Oxalsäure, Zitronensäure, Apfelsäure, Schleimsäure, Benzoesäure, Salicylsäure, Acetursäure, Embonsäure, Naphthalin-1,5-disulfonsäure, Ascorbinsäure, Phenylessigsäure, p-Aminosalicylsäure, Hydroxyäthansulfonsäure, Benzolsulfonsäure oder synthetische Harze, die saure Gruppen enthalten, z. B. solche mit Ionenaustauschwirkung. Als Lösungsmittel der entsprechenden physiologisch verträglichen Salze der aktiven Verbindungen für eine intravenöse Applikation kommen z. B. in Frage: Wasser, physiologische Kochsalzlösungen oder Alkohol, wie Äthanol, Propandiol oder Glycerin, daneben auch Zuckerlösungen, wie z. B. Glukose- oder Mannitlösungen, oder auch eine Mischung aus den verschiedenen genannten Lösungsmitteln.

Beispiel 1
3-N-Methylpiperazino-1-phenyl-isochinolin-4-aldehyd

Ein Gemisch aus 20 g 3-Chlor-1-phenyl-isochinolin-4-aldehyd und 15 g N-Methylpiperazin werden in 200 ml Toluol 4 Stunden am Rückfluss gekocht. Nach dem Abkühlen wird die Toluollösung 4mal mit je 200 ml Wasser gewaschen, mit Magnesiumsulfat getrocknet, filtriert und das Lösungsmittel im Vakuum entfernt. Der Rückstand wird mit Diisopropyläther verrieben und 21,0 g weisse Kristalle mit Schmelzpunkt 152–154° abgesaugt (Hydrochlorid Schmp. 230°).

Wie vorstehend werden die 3-substituierten Isochinolin-4-aldehyde der Beispiele in Tabelle 1 aus den 3-Chlor-isochinolin-4-aldehyden und den entsprechenden Basen dargestellt.

Tabelle 1

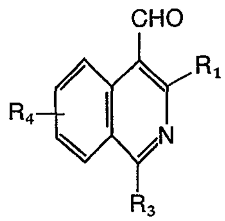

| Beispiel | $R_1$ | $R_3$ | $R_4$ | Schmp. °C, Salz (Schmp. °C) |
|---|---|---|---|---|
| 2 | 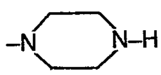 | $C_6H_5$ | H | 147–149°, Hydrochlorid (218°) |
| 3 | 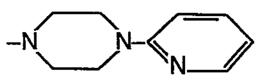 | $C_6H_5$ | H | 174–176° |
| 4 | 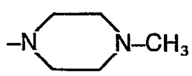 | 2,4-Di-Cl-$C_6H_3$ | H | 166–168°, Hydrochlorid (231°) |
| 5 | 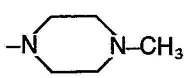 | 4-Cl-$C_6H_4$– | H | 182–184°, Hydrochlorid (230°) |
| 6 | 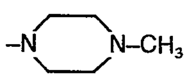 | $C_6H_5$ | 6,7-Di-$CH_3O$ | 158–160°, Hydrochlorid (214°) |
| 7 | 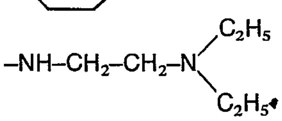 | $C_6H_5$ | 6-Cl | Öl, Hydrochlorid (212°) |
| 8 | 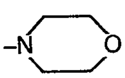 | $C_6H_5$ | H | Hydrochlorid (270°) |
| 9 | 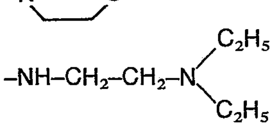 | 4-Cl-$C_6H_4$ | H | Öl, Hydrochlorid (210°) |
| 10 | 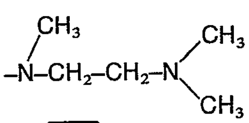 | $C_6H_5$ | H | Öl, Hydrochlorid (195°) |
| 11 | 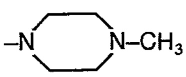 | $C_6H_5$ | 6-Cl | 185–187°, Hydrochlorid (240°) |
| 12 | 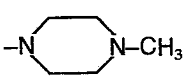 | 2-F-$C_6H_4$ | H | 125–126°, Hydrochlorid (234°) |
| 13 | 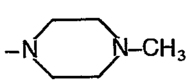 | 2-$CH_3$-$C_6H_4$ | H | 143–146°, Hydrochlorid (235°) |

Tabelle 1 (Fortsetzung)

| Beispiel | $R_1$ | $R_3$ | $R_4$ | Schmp. °C, Salz (Schmp. °C) |
| --- | --- | --- | --- | --- |
| 14 | 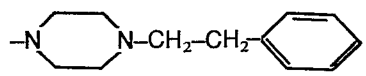 | $C_6H_5$ | H | 146–148°, Hydrochlorid (252°) |
| 15 | 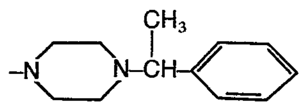 | $C_6H_5$ | H | 166–168°, Hydrochlorid (258°) |
| 16 | 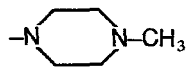 | 4-Pyridyl | H | 181–182° |
| 17 | 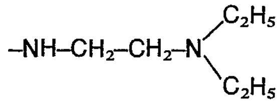 | $C_6H_5$ | H | 73–74° |

Beispiel 18
3-N-Carboäthoxypiperazino-1-phenyl-isochinolin-4-aldehyd

Zu 9,55 g 3-Piperazino-1-phenyl-isochinolin-4-aldehyd Hydrochlorid, 11,5 g Natriumcarbonat in 200 ml Toluol werden bei Raumtemperatur unter Rühren 8,7 g Chlorameisensäureäthylester getropft. Die Mischung wird anschliessend 2 Stunden am Rückfluss gekocht, abgekühlt und vom anorganischen Niederschlag abfiltriert. Das Filtrat wird im Vakuum eingeengt und der Rückstand kristallisiert. Man erhält 8,4 g 3-N-Carboäthoxypiperazino-1-phenyl-isochinolin-4-aldehyd mit Schmp. 138–140 °C.

Beispiel 19
3-N-Butylpiperazino-1-phenyl-isochinolin-4-aldehyd

Analog zur Arbeitsweise von Beispiel 18 erhält man mit Butylbromid öliges 3-N-Butylpiperazino-1-phenyl-isochinolin-4-aldehyd, dessen Hydrochlorid bei 202 °C schmilzt.

Beispiel 20
3-N-Äthylpiperazino-1-phenyl-isochinolin-4-aldehyd

Analog zur Arbeitsweise von Beispiel 18 erhält man mit Äthyljodid gelbliche Kristalle des 3-N-Äthylpiperazino-1-phenyl-isochinolin-4-aldehyd mit Schmp. 136–149 °C.

Beispiel 21
3-N-(2-Furoyl)-piperazino-1-phenyl-isochinolin-4-aldehyd

10,6 g 3-Piperazino-1-phenyl-isochinolin-4-aldehyd Hydrochlorid werden in 300 ml Pyridin bei 0 °C mit 6,44 g Furan-2-carbonsäurechlorid versetzt. Nach 4 Stunden Rühren bei Raumtemperatur wird das Lösungsmittel im Vakuum entfernt und der Rückstand aus Wasser kristallisiert. Man isoliert mit Schmp. 128 °C.

Beispiel 22
4-Hydroxymethyl-3-N-methylpiperazino-1-phenyl-isochinolin

Es werden 10 g 3-N-Methylpiperazino-1-phenyl-isochinolin-4-aldehyd in 300 ml Methanol suspendiert und bei Raumtemperatur mit 3,5 g Natriumborhydrid in kleinen Portionen versetzt. Man rührt noch 4 Stunden bei Raumtemperatur nach, entfernt das Lösungsmittel im Vakuum und verrührt den Rückstand mit 500 ml Wasser. Es können 10,0 g 4-Hydroxymethyl-3-N-methylpiperazino-1-phenyl-isochinolin mit Schmp. 153 bis 155 °C abgesaugt werden. Hydrochlorid 225 °C.

Die in Tabelle 2 aufgeführten Beispiele werden entsprechend dargestellt.

Tabelle 2

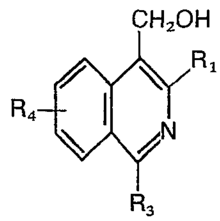

| Beispiel | $R_1$ | $R_3$ | $R_4$ | Schmp. °C, Salz (Schmp. °C) |
|---|---|---|---|---|
| 23 | 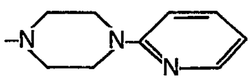 | $C_6H_5$ | H | 179–181° |
| 24 | 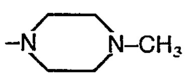 | 2,4-Di-Cl–$C_6H_3$ | H | 109–116°, Hydrochlorid (238°) |
| 25 | 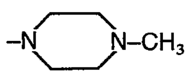 | 4-Cl–$C_6H_4$ | H | 169–172°, Hydrochlorid (238°) |
| 26 | 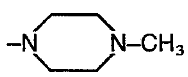 | $C_6H_5$ | 6,7-Di–$CH_3O$ | 164–170°, Hydrochlorid (230°) |
| 27 | 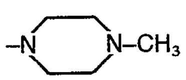 | $C_6H_5$ | 6-Cl | 202–205°, Hydrochlorid (254°) |
| 28 | 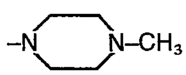 | $C_6H_5$ | 2-F | 140–143°, Hydrochlorid (229°) |
| 29 | 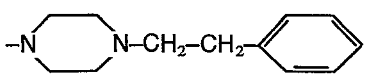 | $C_6H_5$ | H | 142–144°, Hydrochlorid (212°) |
| 30 | 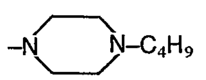 | $C_6H_5$ | H | 102–104° |
| 31 | 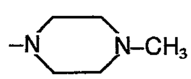 | 3-Cl–$C_6H_4$ | H | 112–114° |
| 32 | 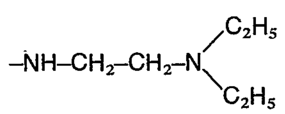 | –$C_6H_5$ | H | 109–110°C |
| 33 | 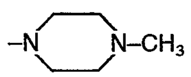 | 4-Pyridyl | H | 210–215° |

Beispiel 34
4-Cyano-3-N-methylpiperazino-1-phenylisochinolin

2,64 g 3-Chlor-4-cyano-1-phenylisochinolin werden mit 2,0 g N-Methylpiperazin in 60 ml Toluol 6 Stunden am Rückfluss gekocht. Nach dem Abkühlen wird die Toluolphase mehrmals mit Wasser gewaschen, getrocknet und das Lösungsmittel im Vakuum entfernt. Der Rückstand wird aus Methanol umkristallisiert. 2,4 g mit Schmp. 254–256 °C. Hydrochlorid 256–258 °C.

Das Ausgangsmaterial kann aus 3-Chlor-1-phenylisochinolin-4-aldehyd folgendermassen dargestellt werden: 53,6 g 3-Chlor-1-phenylisochinolin-4-aldehyd in 150 ml Pyridin wird bei 0 °C mit 55,6 g Hydroxylaminhydrochlorid versetzt und nach 2 Std. bei Raumtemperatur das Lösungsmittel mit Vakuum entfernt. Der Rückstand wird zwischen Wasser und Toluol verteilt. Die Toluolphase wird getrocknet und einrotiert. Man erhält 43,2 g Aldoxim mit Schmp. 151–154 °C. 67,3 g Aldoxim werden in 700 ml Pyridin bei 0 °C mit 91,4 g Phosphoroxychlorid versetzt und dann 12 Std. bei Raumtemperatur stehengelassen. Das Reaktionsgemisch wird mit Wasser hydrolysiert und der kristalline Niederschlag abfiltriert. Nach dem Umlösen aus Äthanol erhält man 47 g 3-Chlor-4-cyano-1-phenylisochinolin mit Schmp. 191–193 °C.

Beispiel 35
4-Cyano-3-N-methylpiperazino-1-phenyl-isochinolin

6,7 g 3-N-Methylpiperazino-1-phenyl-isochinolin-4-aldoxim werden wie oben beschrieben mit Phosphoroxychlorid in Pyridin behandelt. Nach üblicher Aufarbeitung isoliert man 5,1 g 3-N-Methylpiperazino-4-cyano-1-phenyl-isochinolin mit Schmp. 254–256 °C.

Das Ausgangsmaterial wird wie folgt dargestellt:

16,6 g 3-N-Methylpiperazino-1-phenyl-isochinolin-4-aldehyd werden in 150 ml Pyridin mit 13,9 g Hydroxylamin hydrochlorid wie im Beispiel 34 beschrieben, behandelt. Nach üblicher Aufarbeitung isoliert man 17,2 g Aldoxim mit Schmp. 234–235 °C. Hydrochlorid 235 °C.

Beispiel 36
4-Aminomethyl-3-N-methylpiperazino-1-phenyl-isochinolin

9,8 g 3-N-Methylpiperazino-1-phenyl-isochinolin-4-aldoxim werden in 500 ml Methanol und 100 ml 8n-methanolische Ammoniaklösung mit 10 g Raney-Nickel in einer Wasserstoffatmosphäre bei Raumtemperatur geschüttelt. Nachdem die theoretische Menge Wasserstoff aufgenommen ist, filtriert man den Katalysator ab und entfernt das Lösungsmittel im Vakuum. Der Rückstand kristallisiert aus Toluol mit Schmp. 122–125 °C. Hydrochlorid 251 °C.

Beispiel 37
1-Phenyl-3-piperazino-isochinolin-4-carbonsäure-N-methylpiperazid

12,06 g 3-Chlor-1-phenyl-isochinolin-4-carbonsäure-N-methylpiperazid in 50 ml Dioxan werden mit 90 g Piperazin unter Rühren 80 Std. am Rückfluss getrocknet. Die Reaktionslösung wird in gesättigte Natriumchlorid-Lösung eingegossen und mit Toluol extrahiert. Das Toluol wird im Vakuum entfernt und das rotbraune Harz als Hydrochlorid kristallisiert. Man isoliert 14,7 g mit Schmp. 280 °C.

Das eingesetzte Ausgangsmaterial kann wie folgt hergestellt werden:

53,5 g 3-Chlor-1-phenyl-isochinolin-4-aldehyd wird in 1,5 l Aceton und 500 ml Phosphatpuffer vom pH 7 suspendiert. Bei 40 °C trägt man portionsweise im Laufe von 2 Stunden 40 g Kaliumpermanganat ein und rührt bei dieser Temperatur 2 Stunden nach. Das überschüssige Kaliumpermanganat wird mit 10 g Natriumhydrogensulfit zerstört und die Lösung auf 500 ml einrotiert und filtriert. Das Filtrat wird mit konzentrierter Salzsäure auf pH 4 gebracht und mit Essigester gründlich extrahiert. Nach dem Entfernen des Lösungsmittels im Vakuum verbleiben 41,1 g 3-Chlor-1-phenyl-isochinolin-4-carbonsäure mit Schmp. 208 °C.

42,5 g 3-Chlor-1-phenyl-isochinolin-4-carbonsäure werden mit 300 ml Thionylchlorid 4 Std. am Rückfluss gekocht. Die Lösung wird einrotiert und das rohe Säurechlorid sofort weiterverarbeitet.

Zu 11,3 g N-Methylpiperazin in 100 ml Chloroform werden bei Raumtemperatur 11,3 g Säurechlorid in 75 ml Chloroform getropft. Nach 6 Std. bei Raumtemperatur wird das Lösungsmittel entfernt und der Rückstand mit gesättigter Natriumhydrogencarbonatlösung verrührt. Es können 13,3 g des N-Methylpiperazids mit Schmp. 164 bis 167 °C isoliert werden. Hydrochlorid 256 °C.

Gemäss dem Beispiel 37 können die Verbindungen der Tabelle dargestellt werden

Tabelle 3

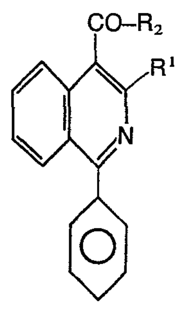

| Beispiel | $R_1$ | $R_2$ | Schmp. °C, Salz (Schmp. °C) |
|---|---|---|---|
| 38 | 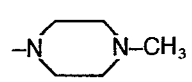 | 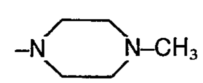 | 156–158°, Dihydrochlorid (290°) |
| 39 | 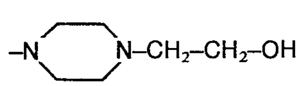 | 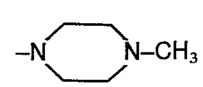 | Öl, Hydrochlorid (273°) |

Tabelle 3 (Fortsetzung)

| Beispiel | $R_1$ | $R_2$ | Schmp. °C, Salz (Schmp. °C) |
|---|---|---|---|
| 40 | 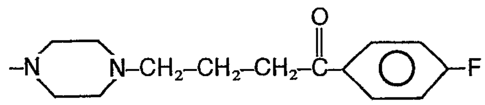 | 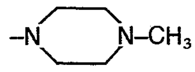 | Öl, hydrochlorid (140°) |
| 41 | 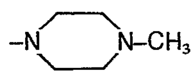 | 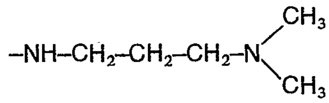 | Öl, Dihydrochlorid (260°) |
| 42 | 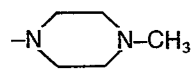 | 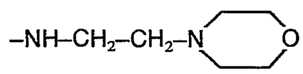 | Öl, Hydrochlorid (170°) |

Beispiel 43
3-(3-N-Methylpiperazino-1-phenyl-isochinolin-4-yl)-acrylsäure-nitril

Unter Stickstoff werden zu 0,6 g Natriumhydrid in 75 ml absoluten Dimethoxyäthan bei Raumtemperatur 4,43 g Cyanomethyldiäthylphosphat getropft. Nachdem 45 Minuten bei Raumtemperatur gerührt wurde, gibt man 8,3 g 3-N-Methylpiperazino-1-phenyl-isochinolin-4-aldehyd gelöst in 100 ml Dimethoxyäthan zu. Man lässt 12 Stunden bei Raumtemperatur stehen, hydrolysiert mit Wasser, entfernt das Lösungsmittel und verteilt den Rückstand zwischen Toluol und Wasser. Aus der Toluolphase isoliert man 6,9 g Acrylsäurenitrilderivat mit Schmp. 166–168 °C. Hydrochlorid 245 °C.

Beispiel 44
4-Äthoxy-methylen-3-N-äthylpiperazino-1-phenyl-isochinolin

3 g 4-Hydroxymethyl-3-N-äthylpiperazino-1-phenyl-isochinolin wird in Äthanol gelöst und mit 10 ml 0,2n äthanolischer Salzsäure auf dem Wasserbad für 1 Std. erhitzt. Das Lösungsmittel wird im Vakuum entfernt und die Kristalle mit Aceton/Äther verrührt. Es verbleiben 2,3 g der 4-Äthoxymethylenverbindung als Hydrochlorid mit Schmp. 186 °C.

Beispiel 45
4-Acetoxymethyl-3-N-methylpiperazino-1-phenyl-isochinolin

3,1 g 4-Hydroxymethylen-3-N-methylpiperazino-1-phenyl-isochinolin wird in 30 ml Pyridin bei Raumtemperatur mit 1,1 g Acetylchlorid versetzt. Nach 4 Std. bei Raumtemperatur wird das Pyridin im Vakuum entfernt und der Rückstand zwischen Methylenchlorid und Wasser verteilt. Aus der Methylenchloridphase isoliert man 1,8 g mit Schmp. 126–128 °C.

Beispiel 46
3-(3-N-Methylpiperazino-1-phenyl-isochinolin-4-yl)-acrylaldehyd

Zu 3,54 g 3-(3-N-Methylpiperazino-1-phenylisochinon-4-yl)-acrylsäure-nitril in 150 ml Toluol werden unter Stickstoffschutzgas bei 0 °C 2,13 g 20%iges Diisobutylaluminiumhydrid in Toluol getropft. Nach 1 Std. Rühren bei 0 °C wird mit Eisessig hydrolysiert und die Toluolphase mit Wasser gewaschen. Nach dem Entfernen des Lösungsmittels im Vakuum verreibt man den öligen Rückstand mit Äther und filtriert 2,8 g mit Schmp. 130–133°.

Beispiel 47
4-(2-Diäthylaminoäthyl)-aminomethyl-3-N-methylpiperazino-1-phenyl-isochinolin

10 g 3-N-Methylpiperazino-1-phenyl-isochinolin-4-aldehyd in 250 ml Methanol wird 10 Std. mit Diäthylaminoäthylamin am Rückfluss gekocht. Das Methanol und Reaktionswasser wird im Vakuum entfernt und der Rückstand in 300 ml frischem Methanol gelöst. Unter Eiskühlung gibt man portionsweise 6,86 g Natriumborhydrid zu und rührt 12 Stunden bei Raumtemperatur. Das Lösungsmittel wird im Vakuum entfernt und der Rückstand zwischen Äther und Wasser verteilt. Aus der Ätherlösung, isoliert man 14,5 g mit Schmp. 95–97 °C Trimaleinat: Schmp. 166°.

Analog zu diesem Beispiel wurden die Verbindungen der Tabelle 4 dargestellt.

Tabelle 4

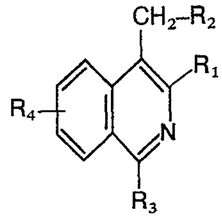

| Beispiel | $R_1$ | $R_2$ | $R_3$ | $R_4$ | Schmp. °C, Salz (Schmp. °C) |
|---|---|---|---|---|---|
| 48 | 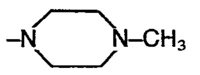 | $-NH-C_4H_9$ | $C_6H_5$ | H | Öl, Dimaleinat (167°) |
| 49 | 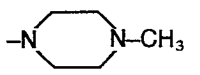 | 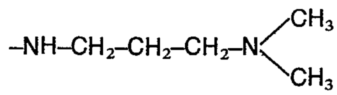 | $C_6H_5$ | H | 74–76°, Trimaleinat (158°) |
| 50 | 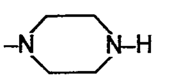 | 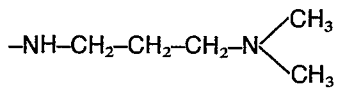 | $C_6H_5$ | H | Öl, Trihydrochlorid (166°) |
| 51 | 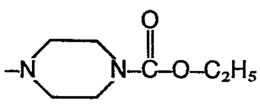 | 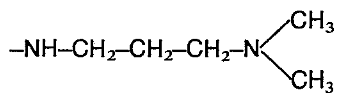 | $C_6H_5$ | H | Öl, Dimaleinat (154°) |
| 52 | 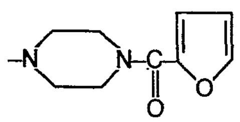 | 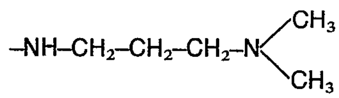 | $C_6H_5$ | H | Öl, Maleinat (132°) |
| 53 | 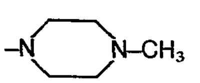 | 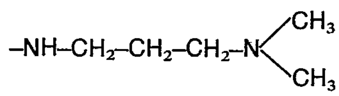 | $2-F-C_6H_4$ | H | 95–96°, Trimaleinat (143°) |
| 54 | 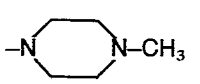 | 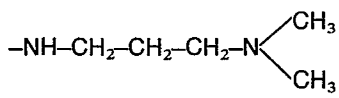 | $C_6H_5$ | 6-Cl | Öl, Trimaleinat (173°) |
| 55 | 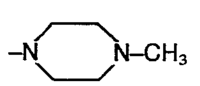 | 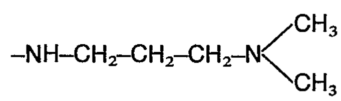 | $2-CH_3-C_6H_4$ | H | Öl, Trioxalat (145°) |

Beispiel 56

3-N-Methylpiperazino-4-(N-(2-methoxyphenyl)-piperazinomethyl)-1-phenyl-isochinolin

4,81 g 4-Hydroxymethyl-3-(N-methylpiperazino)-1-phenyl-isochinolin wird in 200 ml Toluol mit 3,1 g Phosphorpentachlorid bei Raumtemperatur 3 Std. gerührt. Das Lösungsmittel und Phosphoroxychlorid wird im Vakuum entfernt. Dann wird der Rückstand in Toluol aufgenommen und mit 7,15 g 1-(o-Methoxyphenyl)-piperazin 4 Std. am Rückfluss gekocht. Die Toluollösung wird anschliessend mit Wasser gewaschen und eingeengt. Das zurückbleibende Harz wird in 3,4 g kristallines Dihydrochlorid mit Schmp. >290° überführt.

Beispiel 57

3-N-(2-pyridylpiperazino)-1-phenyl-isochinolin-4-aldehyd

Eine Mischung von 7,88 g 4-Hydroxymethyl-3-N-(2-pyridyl-piperazino)-1-phenyl-isochinolin in 150 ml Chloroform wird mit 4 g aktiviertem Mangandioxid bei Raumtemperatur für 6 Std. gerührt. Nach dem Filtrieren wird die Chloroformphase mit gesättigter Natriumchloridlösung gewaschen und das Lösungsmittel im Vakuum entfernt. Der

harzige Rückstand wird aus Essigester umkristallisiert und so 4,2 g mit Schmp. 170–171 °C isoliert.

**Patentansprüche**

1. Isochinoline der Formel I

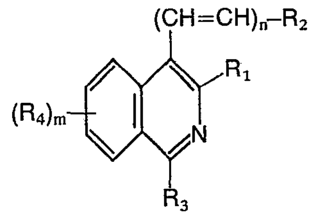

und deren physiologisch verträglichen Salze, worin bedeuten:
m eins oder zwei
n null oder eins
$R_1$ eine Aminogruppe der Formel

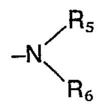 ,

worin $R_5$ und $R_6$ gleich oder verschieden sind und Wasserstoff oder Alkylreste mit 1 bis 8 Kohlenstoffatomen bedeuten, wobei die Alkylreste auch substituiert sein können mit Hydroxy-, $C_1$–$C_4$-Alkoxy oder einer Aminogruppe der Formel

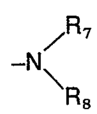 ,

worin $R_7$ und $R_8$ gleich oder verschieden sind und Wasserstoff oder einen geradkettigen oder verzweigten Alkylrest mit 1 bis 6 Kohlenstoffatomen, oder zusammen mit dem Stickstoffatom einen heterocyclischen Ring mit bis zu 7 Kohlenstoffatomen darstellen; die Alkylreste $R_5$ und $R_6$ können auch gemeinsam mit dem Stickstoffatom einen 5- bis 8gliedrigen Ring bilden, wobei der heterocyclische Ring an einem Kohlenstoffatom substituiert sein kann mit einer $C_1$–$C_6$-Alkyl-, $C_1$–$C_4$-Alkoxy-, Hydroxy-, Carboxy- oder $C_1$–$C_4$-Alkoxycarbonylgruppe, und worin eines der Kohlenstoffatome durch ein Sauerstoff-, Schwefel- oder Stickstoffatom ersetzt sein kann, wobei das letztere substituiert sein kann durch Wasserstoff, die Thienyl-, Furyl-, Pyridyl- oder Formylgruppe, eine $C_3$–$C_8$-Alkenyloxycarbonyl- oder $C_3$–$C_8$-Alkinyloxycarbonylgruppe, eine gegebenenfalls durch Hydroxy- oder $C_1$–$C_4$-Alkoxygruppen substituierte $C_1$–$C_6$-Alkoxycarbonylgruppe; durch einen Phenylrest, der einfach oder mehrfach substituiert sein kann mit $C_1$–$C_4$-Alkyl-, $C_1$–$C_4$-Alkoxy, Methylendioxy-, Hydroxy-, Nitro- oder Aminogruppe oder Halogen; durch den Rest $-COR_9$, worin $R_9$ einen Thienyl-, Furyl-, Pyridylrest oder einen gegebenenfalls wie oben angegebenen substituierten Phenylrest; oder durch eine $C_1$–$C_6$-Alkylgruppe, die ihrerseits substituiert sein kann mit Hydroxy, $C_1$–$C_4$-Alkoxy-, $C_1$–$C_6$-Dialkylamino, die Äthylendioxy- oder die Methylendioxygruppe oder einen gegebenenfalls wie oben angegebenen substituierten Phenylrest;
$R_2$ eine Carboxyl-, Cyano, Formyl-, Hydroxymethyl-, eine Alkoxymethylgruppe mit 1 bis 6 Kohlenstoffatomen, eine Aminoalkylgruppe der Formel

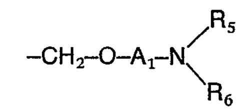 ,

worin $A_1$ eine geradkettige oder verzweigte $C_2$–$C_6$-Alkylengruppe darstellt, die durch Hydroxy oder $C_1$–$C_4$-Alkoxygruppen substituiert sein kann und worin $R_5$ und $R_6$ die oben genannte Bedeutung haben, eine Acyloxymethylgruppe der Formel $-CH_2-O-CO-R_{10}$, worin $R_{10}$ eine $C_1$–$C_6$-Alkyl-, oder einen Phenylrest bedeutet, der gegebenenfalls wie oben angegeben substituiert sein kann, eine Aminomethylgruppe der Formel

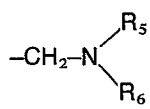 ,

worin $R_5$ und $R_6$ die obengenannte Bedeutung haben, eine Carbonamidgruppe der Formel

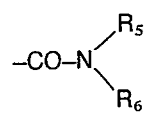 ,

worin $R_5$ und $R_6$ die oben genannte Bedeutung haben, oder eine Carbonestergruppe der Formel

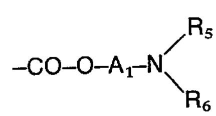 ,

worin $A_1$, $R_5$ und $R_6$ die oben genannte Bedeutung haben,
$R_3$ ein Phenylrest, der gegebenenfalls mit Halogen-, Hydroxy-, Nitro-, Amino- oder einer durch ein oder zwei aliphatische Kohlenwasserstoffreste substituierten Aminogruppe mit zwei bis achtzehn Kohlenstoffatomen, einer Acylamino-, Alkyl- oder Alkoxygruppe mit jeweils einem bis sechs Kohlenstoffatomen, einer Benzyloxy- oder einer Trifluormethylgruppe mono- oder disubstituiert ist, ein Pyridyl- oder Thienylrest;
$R_4$ Wasserstoff, Halogen, Hydroxy-, Alkyl- oder Alkoxygruppe mit einem bis sechs Kohlenstoffatomen, Nitro-, Amino-, Benzyloxy-, Methylendioxy- oder Äthylendioxygruppe.

2. Verfahren zur Herstellung der Verbindungen der Formel I gemäss Anspruch 1, dadurch gekennzeichnet, dass man

$a_1$) Verbindungen der Formel II

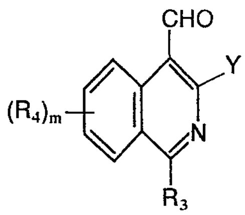

(II)

worin Y Chlor oder Brom und $R_3$, $R_4$ und m die zur Formel I von Anspruch 1 genannte Bedeutung haben, mit einem Amin der Formel

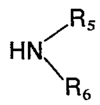

,

worin $R_5$ und $R_6$ die in Anspruch 1 genannte Bedeutung haben, zu Verbindungen der Formel I umsetzt, in denen n gleich Null ist und $R_2$ eine Formylgruppe darstellt, die gegebenenfalls durch Umsetzung mit Verbindungen der Formel IIIa oder IIIb,

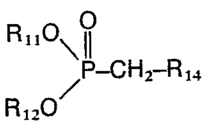

(IIIa)

$(R_{13})_3\overset{\oplus}{P}-CH_2-R_{14}$ $Br^{\ominus}$

(IIIb)

worin $R_{11}$, $R_{12}$ und $R_{13}$ eine $C_1$–$C_4$-Alkylgruppe oder den Phenylrest bedeuten und $R_{14}$ vorzugsweise eine Nitrilgruppe ist, in Verbindungen der Formel I von Anspruch 1 überführt werden können, in denen n = 1 ist;

$a_2$) Verbindungen der Formel IV

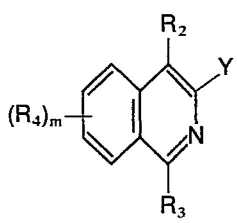

(IV)

worin Y Chlor oder Brom, $R_2$ eine Cyano oder Carbonamidgruppe der Formel

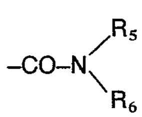

,

und $R_3$, $R_4$, $R_5$, $R_6$ und m die im Anspruch 1 genannte Bedeutung haben, mit einem Amin der Formel

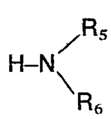

,

worin $R_5$ und $R_6$ die im Anspruch 1 genannte Bedeutung haben, zu Verbindungen gemäss Anspruch 1 umsetzt;

b) Verbindungen der Formel V

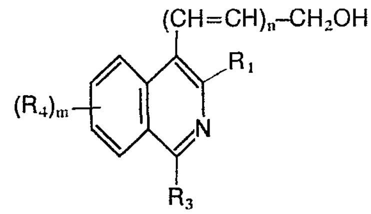

(V)

worin $R_1$, $R_3$, $R_4$, n und m die im Anspruch 1 genannte Bedeutung haben, zu Verbindungen der Formel I von Anspruch 1 oxidiert, worin $R_2$ eine Formylgruppe ist;

c) Verbindungen der Formel VI

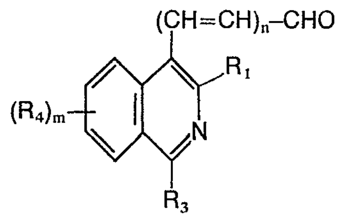

(VI)

worin $R_1$, $R_3$, $R_4$, m und n die im Anspruch 1 genannte Bedeutung haben zu den Verbindungen der Formel I von Anspruch 1 oxidiert, worin $R_2$ eine Carboxylgruppe ist, und diese gegebenenfalls mit einem Amin

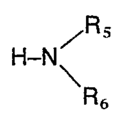

oder mit einem Alkohol der Formel

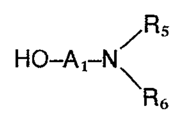

,

worin $R_5$, $R_6$ und $A_1$ die im Anspruch 1 genannte Bedeutung haben, zu Verbindungen gemäss Anspruch 1 umsetzt;

d) Verbindungen der Formel VI zu Verbindungen der Formel I von Anspruch 1 reduziert, worin $R_2$ eine Hydroxymethylgruppe ist;

e) Verbindungen der Formel VII

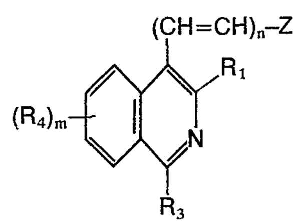

(VII)

worin Z eine Nitril-, Carboxyl-, Halogencarboxyl- oder eine Alkylcarboxylgruppe mit 1 bis 7 Koh-

lenstoffatomen bedeutet und $R_1$, $R_3$, $R_4$, m und n die im Anspruch 1 genannte Bedeutung haben, zu Verbindungen der Formel I reduziert, worin $R_2$ eine Formylgruppe darstellt;
f) Verbindungen der Formel VIII

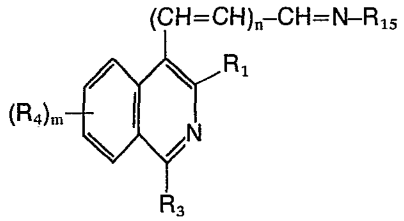

(VIII)

worin $R_1$, $R_3$, $R_4$, m und n die in Anspruch 1 genannte Bedeutung haben, zu Verbindungen der Formel I von Anspruch 1 reduziert, worin $R_2$ die Methylenaminogruppe der Formel $-CH_2-NH-R_{15}$ und $R_{15}$ der Rest

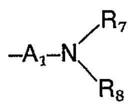

sein soll, und $A_1$, $R_7$ und $R_8$ die in Anspruch 1 genannte Bedeutung haben;
g) Verbindungen der Formel IX

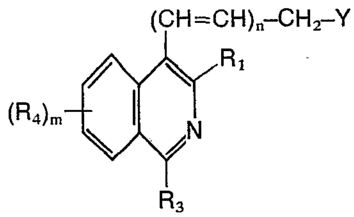

(IX)

worin Y gleich Chlor oder Brom ist mit einem Amin der Formel

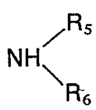

,

oder einem Alkohol der Formel

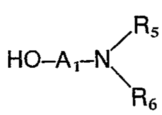

umsetzt, wobei in den obigen Formeln m, n, $R_1$, $R_3$ bis $R_6$ und $A_1$ die im Anspruch 1 genannte Bedeutung haben.
h) Verbindungen der Formel X

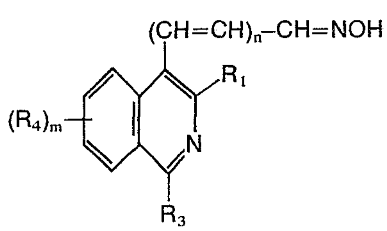

(X)

worin $R_1$, $R_3$, $R_4$, m und n die in Anspruch 1 genannte Bedeutung haben, mit wasserabspaltenden Mitteln in Verbindungen der Formel I von Anspruch 1 überführt, worin $R_2$ eine Nitrilgruppe darstellt;
i) Verbindungen der Formel XI

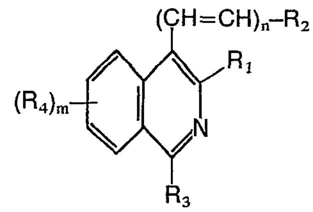

(XI)

worin $R_2$ bis $R_4$, m und n die in Anspruch 1 genannten Bedeutungen haben und $R_1$ ein Piperazinylring ist, mit einem Alkylierungsmittel der Formel $Y-R_{16}$, worin Y Chlor oder Brom und $R_{16}$ einen geradkettigen oder verzweigten $C_1$–$C_6$-Alkylrest bedeuten, der durch Hydroxy, $C_1$–$C_4$-Alkoxy, $C_1$–$C_4$-Dialkylamino, Äthylendioxy, Methylendioxy, Phenyl oder eine oder mehrere $C_1$–$C_4$-Alkyl-, $C_1$–$C_4$-Alkoxy-, Methylendioxy-, Hydroxy-, Nitro- oder Aminogruppen oder Halogen tragendes Phenyl substituiert sein kann, oder mit einem Chlorameisensäureester der Formel $Cl-COO-(C_1-C_4)$-alkyl oder mit einer Verbindung der Formel $Cl-COR_9$, in der $R_9$ die in Anspruch 1 genannten Bedeutungen hat, umsetzt.

3. Arzneimittel, gekennzeichnet durch einen Gehalt an einer Verbindung der Formel I von Anspruch I oder bestehend aus einer solchen Verbindung.

**Claims**

1. Isoquinolines of the formula I

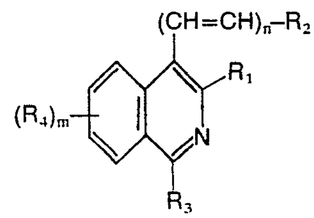

(I)

and their physiologically compatible salts,
in which
m denotes one or two,
n denotes zero or one,
$R_1$ denotes an amino group of the formula

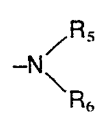

.

in which $R_5$ and $R_6$ are identical or different and denote hydrogen or an alkyl radical with 1 to 8 carbon atoms, it being possible for the alkyl radicals also to be substituted by hydroxyl, $C_1$–$C_4$-alkoxy or an amino group of the formula

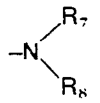

in which $R_7$ and $R_8$ are identical or different and represent hydrogen or a straight-chain or branched alkyl radical with 1 to 6 carbon atoms, or together with the nitrogen atom represent a heterocyclic ring with up to 7 carbon atoms, and the alkyl radicals $R_5$ and $R_6$, together with the nitrogen atom, can also form a 5-membered to 8-membered ring, and the heterocyclic ring can be substituted on one carbon atom by a $C_1$–$C_6$-alkyl, $C_1$–$C_4$-alkoxy, hydroxyl, carboxyl or $C_1$–$C_4$-alkoxycarbonyl group, and in the said ring one of the carbon atoms can be replaced by an oxygen sulfur or nitrogen atom and the latter can be substituted by hydrogen, the thienyl, furyl, pyridyl or formyl group, a $C_3$–$C_8$-alkenyloxycarbonyl or $C_3$–$C_8$-alkynyloxycarbonyl group, a $C_1$–$C_6$-alkoxycarbonyl group which is optionally substituted by hydroxyl or $C_1$–$C_4$-alkoxy groups, or a phenyl radical, which can be monosubstituted or polysubstituted by $C_1$–$C_4$-alkyl, $C_1$–$C_4$-alkoxy, methylenedioxy, hydroxyl, nitro or amino groups or halogen, and the hydrogen atom on the nitrogen can, furthermore, be replaced by the radical $-COR_9$, in which $R_9$ denotes a thienyl, furyl or pyridyl radical or a phenyl radical which is optionally substituted as indicated above, or denotes a $C_1$–$C_6$-alkyl group, which in turn can be substituted by hydroxyl or a $C_1$–$C_4$-alkoxy, $C_1$–$C_4$-dialkylamino, ethylenedioxy or methylendioxy group or a phenyl radical which is optionally substituted as indicated above; $R_2$ denotes a carboxyl, cyano, formyl or hydroxymethyl group, an alkoxymethyl group with 1 to 6 carbon atoms, an aminoalkyl group for the formula

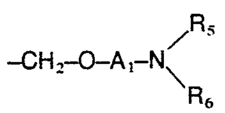

in which $A_1$ represents a straight-chain or branched $C_2$–$C_6$-alkylene group, which can be substituted by hydroxyl or $C_1$–$C_4$-alkoxy groups, and in which $R_5$ and $R_6$ are as defined above, an acyloxymethyl group of the formula $-CH_2-O-CO-R_{10}$, in which $R_{10}$ is a $C_1$–$C_6$-alkyl radical or a phenyl radical, which optionally can be substituted as indicated above, an aminomethyl group of the formula

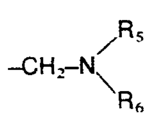

in which $R_5$ and $R_6$ are as defined above, a carboxamide group of the formula

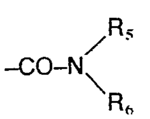

in which $R_5$ and $R_6$ are as defined above, or a carboxylic acid ester group of the formula

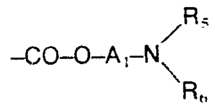

in which $A_1$, $R_5$ and $R_6$ are as defined above; $R_3$ denotes a phenyl radical, which optionally is monosubstituted or disubstituted by halogen, hydroxyl, nitro, amino or an amino group substituted in turn by one or two alkyl radicals having two to eighteen carbon atoms, or by an acylamino, alkyl or alkoxy group, each with one to six carbon atoms, a benzyloxy group or a trifluoromethyl group, or denotes a pyridyl or thienyl radical; and $R_4$ denotes hydrogen, halogen, hydroxyl, an alkyl or alkoxy group with one to six carbon atoms, or a nitro, amino,benzyloxy or methylenedioxy or ethylenedioxy group.

2. Process for the manufacture of the compounds of the formula I in claim 1, which comprises

$a_1$) reacting compounds of the formula II

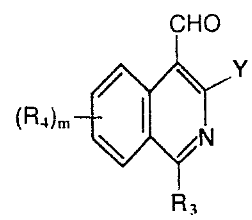

(II)

in which Y denotes chlorine or bromine and $R_3$, $R_4$ and m are as defined in claim 1, with an amine of the formula

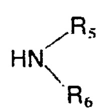

in which $R_5$ and $R_6$ are as defined in claim 1, to give the compounds, according to the invention, of the formula I, in which n is zero and $R_2$ represents a formyl group, it being possible to convert these compounds, if desired, to compounds of the formula I in which n is 1 by reaction with compounds of the formula IIIa or IIIb

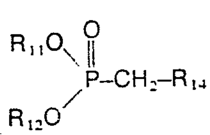

$(R_{13})_3\overset{\oplus}{P}-CH_2-R_{14}\ \ Br^{\ominus}$

(IIIa) (IIIb)

in which $R_{11}$, $R_{12}$ and $R_{13}$ denote a $C_1$–$C_4$-alkyl group or the phenyl radical and $R_{14}$ is preferably a nitrile group,

$a_2$) reacting compounds of the formula IV

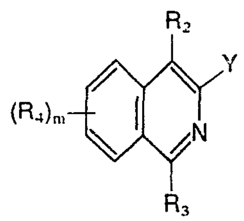

(IV)

in which Y denotes chlorine or bromine, $R_2$ denotes a cyano group or a carboxamide group of the formula

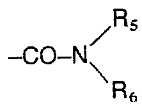
,

and $R_3$, $R_4$, $R_5$, $R_6$ and m are as defined in claim 1, with an amine of the formula

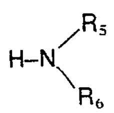
,

in which $R_5$ and $R_6$ are as defined in claim 1, to give the compounds, according to the invention, of the formula I,

b) oxidizing compounds of the formula V

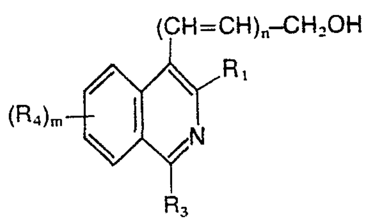

(V)

in which $R_1$, $R_3$, $R_4$, n and m are as defined in claim 1, to compounds of the formula I in claim 1 in which $R_2$ is a formyl group,

c) oxidizing compounds of the formula VI

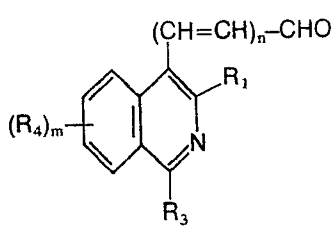

(VI)

in which $R_1$, $R_3$, $R_4$, m and n are as defined in claim 1, to compounds of the formula I in claim 1, in which $R_2$ is a carboxyl group, it being possible, if desired, to convert these compounds to compounds of the formula I, by reaction with an amine

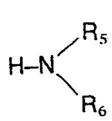

or with an alcohol of the formula

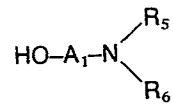
,

in which $R_5$, $R_6$ and $A_1$ are as defined in claim 1,

d) reducing compounds of the formula VI to compounds of the formula I in which $R_2$ is a hydroxyl group,

e) reducing compounds of the formula VII

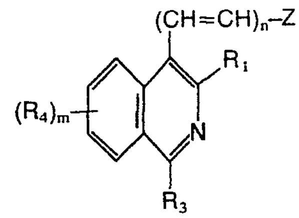

(VII)

in which Z denotes a nitrile, carboxyl or halogenocarboxyl group or an alkylcarbonyl group with 1 to 7 carbon atoms and $R_1$, $R_3$, $R_4$, m and n are as defined in claim 1, to compounds of the formula I in which $R_2$ is a formyl group,

f) reducing compounds of the formula VIII

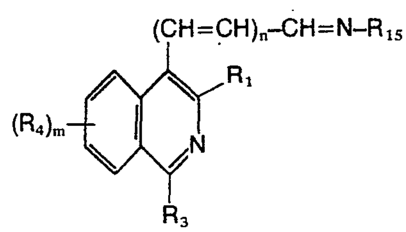

(VIII)

in which $R_1$, $R_3$, $R_4$, m and n are as defined in claim 1, to compounds of the formula I in which $R_2$ represents a methyleneamino group of the formula $-CH_2-NH-R_{15}$ and $R_{15}$ is to be the radical

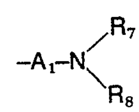

in which $A_1$, $R_7$ and $R_8$ are as defined in claim 1,

g) reacting compounds of the formula IX

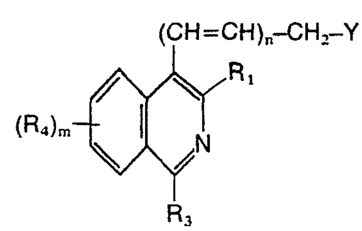

(IX)

in which Y is chlorine or bromine, and $R_1$, $R_3$, $R_4$, m and n are as defined in claim 1, with an amine of the formula

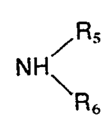
,

or an alcohol of the formula

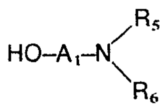

in which $A_1$, $R_5$ and $R_6$ are as defined in claim 1,
h) converting compounds of the formula X

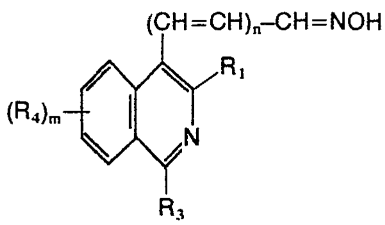

(X)

in which $R_1$, $R_3$, $R_4$, m and n are as defined in claim 1, by means of dehydrating agents to compounds of the formula I in which $R_2$ is a nitrile group,
i) reacting compounds of the formula XI

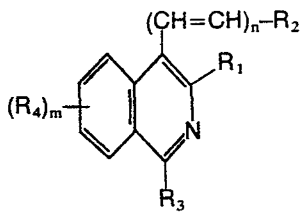

(XI)

in which $R_2$ to $R_4$, m and n are as defined in claim 1, and $R_1$ denotes a piperazine ring, with an alkylating agent of the formula $Y-R_{16}$, in which Y denotes chlorine or bromine and $R_{16}$ denotes a straight-chain or branched $C_1-C_6$-alkyl radical, which can be substituted by hydroxyl, $C_1-C_4$-alkoxy, $C_1-C_4$-dialkylamino, ethylenedioxy, methylenedioxy or phenyl being optionally substituted by one or more $C_1-C_4$-alkyl, $C_1-C_4$-alkoxy, methylenedioxy, hydroxy, nitro or amino groups or halogen atoms, or with a chloroformic acid ester of the formula $Cl-COO-(C_1-C_4)$-alkyl, or with a compound of the formula $Cl-COR_9$, in which $R_9$ is as defined in claim 1.

3. Medicaments, which contain a compound of formula I as claimed in claim 1 or consist of such a compound.

**Revendications**

1. Isoquinoléines de formule I

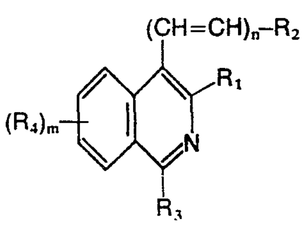

et leurs sels physiologiquement acceptables, formule dans laquelle
m est égal à 1 ou 2,
n est égal à 0 ou 1,
$R_1$ est un groupe amino de formule

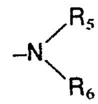

,

dans laquelle $R_5$ et $R_6$ sont identiques ou différents, et désignent l'hydrogène ou des radicaux alkyle de 1 à 8 atomes de carbone, les radicaux alkyle pouvant aussi être substitués par un groupe hydroxy, alcoxy en $C_1$ à $C_4$ ou un groupe amino de formule

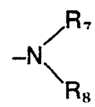

,

dans laquelle $R_7$ et $R_8$ sont identiques ou différents, et désignent l'hydrogène ou un radical alkyle linéaire ou ramifié de 1 à 6 atomes de carbone, ou forment avec l'atome d'azote un noyau hétérocyclique ayant jusqu'à 7 atomes de carbone; les radicaux alkyle $R_5$ et $R_6$ peuvent aussi former avec l'atome d'azote un noyau à 5 à 8 chaînons, le noyau hétérocyclique pouvant être substitué sur un atome de carbone par un groupe alkyle en $C_1$ à $C_6$, alcoxy en $C_1$ à $C_4$, hydroxy, carboxy ou $C_1-C_4$-alcoxycarbonyle, et un des atomes de carbone pouvant être remplacé par un atome d'oxygène, de soufre ou d'azote, ce dernier pouvant être substitué par l'hydrogène, les groupes thiényle, furyle, pyridyle ou formyle, un groupe $C_3-C_8$-alcényloxycarbonyle ou $C_1-C_8$-alcynyloxycarbonyle, un groupe $C_1-C_6$-alcoxycarbonyle éventuellement substitué par des groupes hydroxy ou alcoxy en $C_1$ à $C_4$, par un radical phényle, qui peut être mono- ou polysubstitué par des groupes alkyle en $C_1$ à $C_4$, alcoxy en $C_1$ à $C_4$, méthylènedioxy, hydroxy, nitro ou amino ou des halogènes, par le radical $-COR_9$, où $R_9$ désigne un radical thiényle, furyle, pyridyle ou un radical phényle éventuellement substitué comme il a été indiqué ci-dessus, ou par un groupe alkyle en $C_1$ à $C_6$, qui peut de son côté être substitué par un groupe hydroxy, alcoxy en $C_1$ à $C_4$, $C_1-C_6$-dialkylamino, les groupes éthylênedioxy ou méthylènedioxy ou un radical phényle éventuellement substitué comme il a été indiqué ci-dessus;
$R_2$ désigne un groupe carboxyle, cyano, formyle, hydroxyméthyle, un groupe alcoxyméthyle de 1 à 6 atomes de carbone, un groupe aminoalkyle de formule

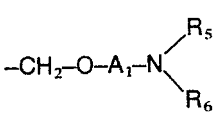

,

dans laquelle $A_1$ désigne un groupe alkylène en $C_2$ à $C_6$ à chaîne droite ou ramifiée, qui peut

être substitué par des groupes hydroxy ou alcoxy en $C_1$ à $C_4$, et dans laquelle $R_5$ et $R_6$ ont la signification indiquée ci-dessus, un groupe acyloxyméthyle de formule $-CH_2-O-CO-R_{10}$, dans laquelle $R_{10}$ désigne un radical alkyle en $C_1$ à $C_6$ ou phényle, qui peut éventuellement être substitué comme il a été indiqué ci-dessus, un groupe aminométhyle de formule

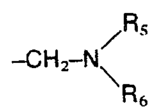
,

dans laquelle $R_5$ et $R_6$ ont la signification indiquée ci-dessus, un groupe carbonamide de formule

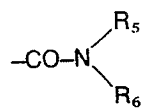
,

dans laquelle $R_5$ et $R_6$ ont la signification indiquée ci-dessus, ou un groupe carbonester de formule

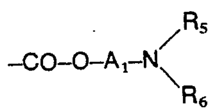
,

dans laquelle $A_1$, $R_5$ et $R_6$ ont la signification indiquée ci-dessus;

$R_3$ désigne un radical phényle, qui est le cas échéant mono- ou disubstitué par halogène, hydroxy, nitro ou amino ou par des groupes amino substitués par un ou deux radicaux hydrocarbonés aliphatiques de 2 à 18 atomes de carbone, un groupe acylamino, alkyle ou alcoxy avec chacun de 1 à 6 atomes de carbone, un groupe benzyloxy ou un groupe trifluorméthyle, ou un radical pyridyle ou thiényle;

$R_4$ désigne l'hydrogène, un halogène, un groupe hydroxy, un groupe alkyle ou alcoxy de 1 à 6 atomes de carbone, un groupe nitro, amino, benzyloxy, méthylènedioxy ou éthylènedioxy.

2. Procédé de préparation des composés de formule I suivant la revendication 1, caractérisé en ce que

$a_1$) On fait réagir des composés de formule II

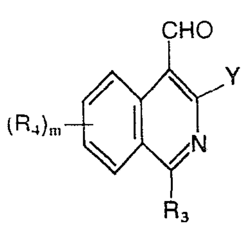
(II)

dans laquelle Y désigne le chlore ou le brome et $R_3$, $R_4$ et m ont la signification indiquée à propos de la formule I de la revendication 1, avec une amine de formule

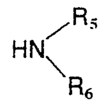
,

dans laquelle $R_5$ et $R_6$ ont la signification indiquée dans la revendication 1, pour donner des composés de formule I dans lesquels n est égal à O et $R_2$ désigne un groupe formyle, qui peuvent le cas échéant être transformés, par réaction avec des composés de formule IIIa ou IIIb

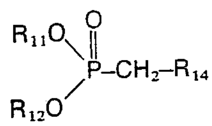
(IIIa)

$(R_{13})_3\overset{\oplus}{P}-CH_2-R_{14}\quad Br^{\ominus}$
(IIIb)

dans lesquelles $R_{11}$, $R_{12}$ et $R_{13}$ désignent un groupe alkyle en $C_1$ à $C_4$ ou le radical phényle et $R_{14}$ est de préférence un groupe nitrile, en composés de formule I suivant la revendication 1, dans lesquels n = 1;

$a_2$) On fait réagir des composés de formule IV

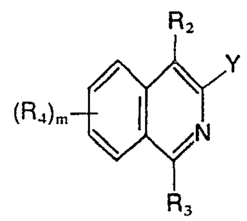
(IV)

dans laquelle Y désigne le chlore ou le brome, $R_2$ est un groupe cyano ou carbonamide de formule

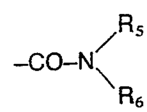
,

et $R_3$, $R_4$, $R_5$, $R_6$ et m ont la signification indiquée dans la revendication 1, avec une amine de formule

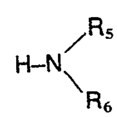
,

dans laquelle $R_5$ et $R_6$ ont les significations indiquées dans la revendication 1, pour donner des composés suivant la revendication 1;

b) On oxyde des composés de formule V

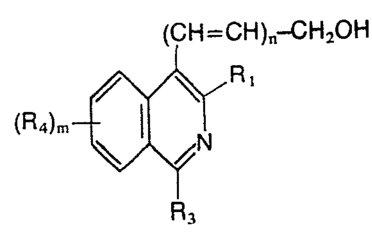
(V)

dans laquelle $R_1$, $R_3$, $R_4$, n et m dont les significations indiquées dans la revendication 1, en composés de formule I de la revendication 1, dans lesquels $R_2$ est un groupe formyle;

c) On oxyde des composés de formule VI

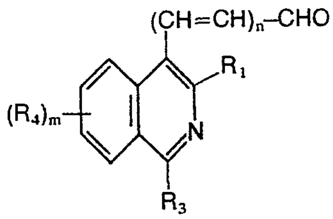

(VI)

dans laquelle $R_1$, $R_3$, $R_4$, m et n ont la signification indiquée dans la revendication 1, en composés de formule I de la revendication 1, dans lesquels $R_2$ est un groupe carboxyle, que l'on fait réagir le cas échéant avec une amine

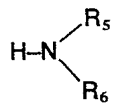

ou avec un alcool de formule

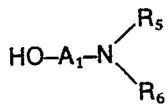

,

$R_5$, $R_6$ et $A_1$ ayant la signification indiquée dans la revendication 1, pour donner des composés suivant la revendication 1;

d)On réduit des composés de formule VI en composés de formule I de la revendication 1, dans lesquels $R_2$ est un groupe hydroxyméthyle;

e) On réduit des composés de formule VII

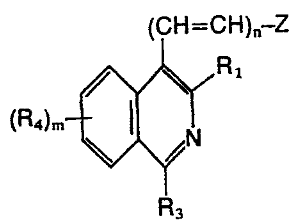

(VII)

dans laquelle Z désigne un groupe nitrile, carboxyle, halogénocarboxyle ou un groupe alkylcarboxyle de 1 à 7 atomes de carbone, et $R_1$, $R_3$, $R_4$, m et n ont la signification indiquée dans la revendication 1, en composés de formule I, dans lesquels $R_2$ représente un groupe formyle;

f) On réduit des composés de formule VIII

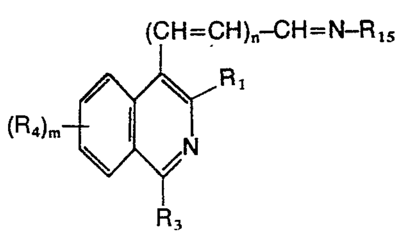

(VIII)

dans laquelle $R_1$, $R_3$, $R_4$, m et n ont la signification indiquée dans la revendication 1, en composés de formule I suivant la revendication 1, dans lesquels $R_2$ doit être le groupe méthylènamino de formule $-CH_2-NH-R_{15}$, et $R_{15}$ doit être le radical

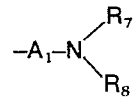

et $A_1$, $R_7$ et $R_8$ ont la signification indiquée dans la revendication 1;

g) On fait réagir des composés de formule IX

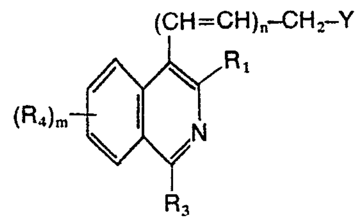

(IX)

dans laquelle Y est le chlore ou le brome, avec une amine de formule

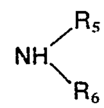

,

ou un alcool de formule

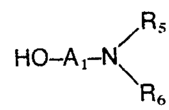

m, n, $R_1$, $R_3$ à $R_6$ et $A_1$ ayant la signification indiquée dans la revendication 1;

h) On transforme des composés de formule X

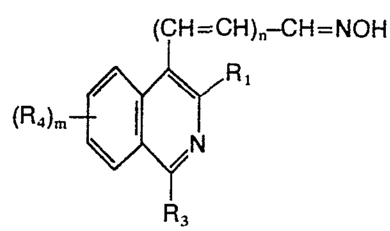

(X)

dans laquelle $R_1$, $R_3$, $R_4$, m et n ont la signification indiquée dans la revendication 1, avec des agents d'élimination de l'eau, en composés de formule I de la revendication 1, dans lesquels $R_2$ désigne un groupe nitrile;

i) On fait réagir des composés de formule XI

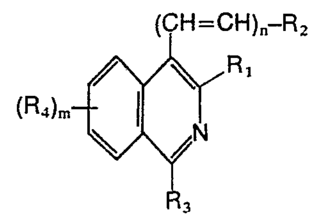

(XI)

dans laquelle $R_2$ à $R_4$, m et n ont la signification indiquée dans la revendication 1, et $R_1$ est un cycle pipérazinyle, avec un agent d'alkylation de formule $Y-R_{16}$, dans laquelle Y désigne le chlore ou le brome et $R_{16}$ représente un radical alkyle en $C_1$ à $C_6$, linéaire ou ramifié, qui peut être substitué par des groupes hydroxy, alcoxy en $C_1$ à $C_4$, $C_1$–$C_4$-dialkylamino, éthylènedioxy, méthylènedioxy, phényle ou phényle portant un ou plusieurs groupes alkyle en $C_1$ à $C_4$, alcoxy en $C_1$ à $C_4$, méthylènedioxy, hydroxy, nitro ou amino ou halogène ou avec un ester chloroformique de formule Cl–COO– (alkyle en $C_1$ à $C_4$) ou avec un composé de formule $Cl-COR_9$, dans laquelle $R_9$ a la signification indiquée dans la revendication 1.

3. Médicament caractérisé en ce qu'il contient un composé de formule I de la revendication 1 ou est constitué d'un tel composé.